# EUROPÄISCHE PATENTANMELDUNG

(11) **EP 1 293 573 A2**
(43) Veröffentlichungstag der Anmeldung: **19.03.2003**
(21) Anmeldenummer: 02018214.3
(22) Anmeldetag: 20.08.2002
(51) Int. Cl.: C12N 15/82, A01H 5/00, C07K 14/18

(54) **Verfahren zum Herstellen eines Markerimpfstoffs gegen ein Säugervirus**

(30) Priorität: 18.09.2001 DE 10145969
(71) Anmelder: Maltagen Forschung GmbH, 86626 Andernach (DE)
(72) Erfinder: Stahl, Rainer, 53179 Bonn (DE); Nelsen-Salz, Birgit, Dr., 50933 Köln (DE); Wolf, Norbert, Dr., 56626 Andernach (DE)
(74) Vertreter: Grünecker, Kinkeldey, Stockmair & Schwanhäusser Anwaltssozietät

(57) **Zusammenfassung**

Die vorliegende Erfindung betrifft ein Verfahren zum Herstellen eines Markerimpfstoffes gegen ein Säugervirus sowie transgene Getreidepflanzen oder Teile davon, die Bestandteile des Impfstoffs herstellen. Insbesondere wird ein Impfstoff zum Bekämpfen der klassischen Schweinepest bereitgestellt.

## Beschreibung

Die vorliegende Erfindung betrifft ein Verfahren zum Herstellen eines Markerimpfstoffs gegen ein Säugervirus sowie transgene Getreidepflanzen oder Teile davon, die Bestandteile des Impfstoffs herstellen.

Virale Infektionen bei Rindern, Schweinen, Hühnern und anderen zur Fleischerzeugung genutzter Tiere verursachen regelmäßig sehr hohe wirtschaftliche Verluste in der Landwirtschaft, da gegen eine Vielzahl viraler Infektionskrankheiten der Tiere keine oder nur unzureichende therapeutische Heilmethoden bzw. effiziente Impfstoffe zur Verfügung stehen. Allein in den Jahren 1995-2000 mussten in Deutschland über eine Million Schweine "gekeult" werden, um die Tilgung der Klassischen Schweinepest (KSP) zu erreichen. In der Europäischen Gemeinschaft werden die wirtschaftlichen Verluste infolge KSP im Zeitraum 1993 - 1997 auf 5 Mrd. ECU bezifferte.

KSP tritt jedoch nicht nur bei Hausschweinen, sondern auch bei unseren einheimischen Wildschweinen auf. Diese Tierart ist seit Anfang der 90er Jahre wieder verstärkt von der Seuche betroffen. Mit dem Virus der KSP infizierte Wildschweine bildeten in den zurückliegenden Jahren zudem eine wichtige Infektionsquelle für unsere Hausschweinebestände. 55% der Ausbrüche an KSP bei Hausschweinen im Zeitraum 1993-1999 werden auf direkte und indirekte Kontakte mit infiziertem Schwarzwild zurückgeführt. Die Bekämpfung der KSP im Schwarzwildbestand ist daher notwendig, um diese Infektionsquelle für unsere Hausschweinebestände auszuschalten.

Zum nachhaltigen Gesundheitsschutz unserer Nutztiere - und nicht zuletzt, um Handelsvorteile zu erreichen - wird von der Veterinärmedizin und der Landwirtschaft die Eradikation gefährlicher Tierseuchen angestrebt. Daher ist für die Landwirte und die Tierärzteschaft die Entwicklung von Impfstoffen für Tiere, die vorbeugend oder aber im Seuchenfall zur Verhinderung bzw. Minimierung einer Weiterverbreitung von Infektionskrankheiten eingesetzt werden können, zu einer zentralen Forderung geworden.

Der bis zum Impfverbot 1992 beim Hausschwein eingesetzte Lebendimpfstoff gegen KSP hat mit dazu beigetragen, dass die Seuche im europäischen Raum zurückgedrängt werden konnte und große wirtschaftliche Schäden in der Landwirtschaft ausblieben. Die Internationalisierung des Marktes machte es aber erforderlich, Impfungen gegen die Klassischen Tierseuchen einzustellen, damit lebende Tiere und Fleischprodukte in und aus der Europäischen Union uneingeschränkt gehandelt werden können. Hintergrund ist, dass geimpfte Tiere und Virusträger serologisch nicht unterschieden werden können. Im letzten Jahr wurde eine neue Impfstoffgeneration - sogenannte Marker-Vakzine - zur Vakzination von Hausschweinen gegen KSP zur Zulassung angemeldet. Für dieses Impfverfahren sollen die rekombinanten viralen Proteine im Baculovektor-Virus Expressionssystem hergestellt werden (EP-A-0 924 298); Tiere entwickeln nach einer Pestivirus-Infektion Antikörper (AK) gegen E^{rns}, E2 (beides Hüllproteine) und NS3 (eine Serin-Protease), von denen nur die AK gegen E2 starke virus-neutralisierende Eigenschaften haben. Nach Verabreichung der Marker-Vakzine bildet das Schwein nur AK gegen das in der Vakzine enthaltene Hüllprotein und durch Anwendung eines spezifischen diagnostischen Testes ist es dann möglich, geimpfte und infizierte Schweine zu unterscheiden. Die Herstellung und vor allem die Applikation - jedes Schwein muss zweimal vakziniert werden - sind jedoch sehr zeit-, arbeits- und kostenintensiv. Diese Vakzine ist zudem nicht oral zu verabreichen, so dass deren Einsatz in der Schwarzwildpopulation nicht möglich ist.

Der vorliegenden Erfindung lag das technische Problem zugrunde, einen weiteren Weg zur Herstellung von Markervakzinen anzugeben, der die Nachteile aus dem Stand der Technik nicht aufweist. Ferner bestand ein technisches Problem darin, die für das genannte Verfahren erforderlichen Mittel bereitzustellen.

Die genannten technischen Probleme werden erfindungsgemäß gelöst durch ein Verfahren zum Herstellen eines Markerimpfstoffs gegen ein Säugervirus, das dadurch gekennzeichnet ist, dass das bzw. die immunogenen Proteine des Impfstoffs von einer Getreidepflanze oder Teilen davon hergestellt werden.

Das weitere technische Problem wird durch die Bereitstellung einer transgenen Getreidepflanze oder Teilen davon, enthaltend mindestens ein Gen, das für ein immunogenes Protein eines Säugervirus kodiert, gelöst.

Die Herstellung von heterologen Produkten, insbesondere heterologen Polypeptiden, in transgenem Pflanzenmaterial hat in den letzten Jahren stark an Bedeutung gewonnen. Neben der Generierung von transgenen Pflanzen, in denen bestimmte Proteine über- oder unterexprimiert werden, mit dem Ziel, verbesserte Pflanzenprodukte durch eine Modifizierung des pflanzlichen Metabolismus zu erhalten, wurde gezielt versucht, transgenes Pflanzenmaterial zur Produktion heterologer Produkte zu konstruieren, die normalerweise nicht in Pflanzen vorkommen, z. B. bakterielle Toxine, menschliche Proteine oder Bioplastik.

Unter dem Begriff "Markerimpfstoff" wird ein Impfstoff verstanden, der zu einer Immunisierung im immunisierten Organismus führt, die sich von der Immunisierung des Organismus durch den eigentlichen Erreger unterscheidet. In der Regel erzeugt der Markerimpfstoff ein anderes Muster an Antikörpern als der eigentliche Erreger. Somit lässt sich anhand der erhaltenen Immunantwort unterscheiden, ob ein Organismus durch einen Markerimpfstoff oder mit dem natürlichen Erreger konfrontiert worden ist.

Eine "Getreidepflanze" im Sinne der vorliegenden Erfindung ist eine Pflanze, die in der Regel auf Grund einer gentechnischen Veränderung des Erbguts genetische Information enthält, die für mindestens ein immunogenes Protein - oder mindestens immunogene Teile davon - kodiert, die Bestandteil des Säugervirus sind, gegen das eine Immunisierung in einem Säugerorganismus gewünscht ist. Das immunogene Protein wird von der Getreidepflanze exprimiert, gegebenenfalls erst nach spezifischer Induktion des Promotors, der die Expression des immunogenen Proteins reguliert.

Das immunogene Protein kann gegebenenfalls auch nur in "Teilen" der Getreidepflanze exprimiert werden. Dies wird regelmäßig der Fall sein, wenn das Gen, das für das immunogene Protein kodiert, unter der Kontrolle eines gewebespezifischen Promotors steht. Demzufolge wird unter "Teilen" der Getreidepflanze z. B. Samen, Blätter oder aber auch Gewebekulturen von Getreidepflanzen verstanden.

Bei einer bevorzugten Ausführungsform wird ein Satz Gene in der Pflanze exprimiert, der einen Teil der immunogenen Proteine (oder besser immunogenen Epitope), aber nicht sämtliche immunogenen Proteine (Epitope) des Säugervirus kodiert. Dies führt dazu, dass die Organismen, die mittels Markerimpfstoff immunisiert worden sind, ein anderes Antikörpermuster erzeugen, als die Organismen, die mit dem natürlichen Erreger infiziert worden sind. Der Markerimpfstoff lässt sich auch durch Zusatz einer immunogenen Komponente modifizieren, so dass die zugesetzte Komponente zu einer zusätzlichen Immunantwort führt, die der natürliche Erreger nicht hervorruft. Eine solche Komponente kann beispielsweise ein Protein sein, das in dem natürlichen Erreger nicht vorkommt, dem Markerimpfstoff aber hinzugefügt wird und somit in dem behandelten Organismus ebenfalls zu einem Antikörpermuster führt, das sich von dem durch die natürlichen Erreger erzeugten Antikörpermuster unterscheidet. Ein solches zusätzliches Protein bzw. immunogene Epitope davon kann beispielsweise als Fusionspartner mit dem eigentlichen immunogenen Protein vorliegen.

Bei einer weiteren bevorzugten Ausführungsform handelt es sich bei der Getreidepflanze um Gerste, Weizen, Roggen, Triticale, Reis bzw. Mais. Insbesondere sind solche Getreidepflanzen bevorzugt, die sich zur Verfütterung an Tiere eignen wie z. B. Weizen, Gerste, Mais.

Bei einer weiteren bevorzugten Ausführungsform ist der Markerimpfstoff zum Immunisieren gegen ein Virus aus der Familie der Pestiviren geeignet, wobei insbesondere eine Immunisierung gegen das Virus der Klassischen Schweinepest, das Border Disease Virus bzw. das Virus der Bovinen Viralen Diarrhoe erfolgt.

Bei einer weiteren bevorzugten Ausführungsform wird das immunogene Protein ausgewählt aus dem Genprodukt des E2-Gens bzw. E^{ms}-Gens des Virus der Klassischen Schweinepest. Es können auch Derivate der genannten Genprodukte eingesetzt werden, sofern sie noch eine ausreichende Immunisierung gegen das genannte Virus hervorrufen können. So kann es sich bei dem Derivat auch um verkürzte Formen des vollständigen Proteins handeln, sofern zumindest einige der immunogenen Epitope erhalten bleiben. Auch kann das Derivat ein Fusionsprotein des E2- bzw. E^{ms}-Genprodukts mit einem weiteren Protein sein, wobei das weitere Protein als der eigentliche "Marker" dient, wie oben erläutert. Auch kann es sich bei dem Derivat um Mutanten des genannten Proteinprodukts halten, die beispielsweise dahingehend optimiert wurden, dass sie eine optimale Immunantwort hervorrufen oder aber die Stabilität des Proteins z. B. in der Getreidepflanze verbessert ist.

Bei einer weiteren bevorzugten Ausführungsform wurden die Kodons des bzw. der Gene dahin optimiert, dass sie optimal für die Getreidepflanze sind, in der der Markerimpfstoff hergestellt werden soll. Dies ermöglicht eine maximale Expression des gewünschten Proteins in der Getreidepflanze. Ein möglichst hoher Gehalt an Protein ist auch hinsichtlich der Immunisierungseigenschaften des aus den Pflanzen hergestellten Markerimpfstoffs wünschenswert.

Bei einer weiteren bevorzugten Ausführungsform erfolgt die Expression des immunogenen Proteins nur in Teilen der Pflanze, wie beispielsweise den Samen, wobei insbesondere Promotoren Verwendung finden, die ausschließlich entweder während der Keimung oder der Kornfüllung aktiv sind. Solche Expressionssysteme sind beispielsweise in der US 5,693,506 sowie WO 97/32986 beschrieben. Dieser Ansatz erlaubt es, das immunogene Protein nicht bereits auf dem Feld sondern erst während der Keimung in einem geschlossenen Produktionsraum in der Pflanze bzw. den Samen zu synthetisieren. Diese sogenannte Mälzungstechnologie befasst sich mit der Keimung von Pflanzenmaterial unter künstlichen Bedingungen. Gewöhnlich wird diese Technologie während der Produktion von Bier angewandt und umfasst Schritte, wie Weichen des Pflanzenmaterials und anschließende Keimung unter kontrollierten Bedingungen. Bei diesen "kontrollierten" Bedingungen werden Parameter wie Feuchtigkeit, Temperatur, Salzkonzentrationen und Bewegung des Pflanzenmaterials durch menschliche Intervention kontrolliert. Solche Prozesse sind z. B. beschrieben in "Die Bierbrauerei" Band 1, "Die Technologie der Malzzubereitung" Ferdinand Enke-Verlag Stuttgart (1999).

Die vorliegende Erfindung betrifft auch eine transgene Getreidepflanze oder deren Teile, enthaltend mindestens ein Gen, das für ein immunogenes Protein eines Säugervirus kodiert, wobei das Gen ein sogenanntes Transgen darstellt. Solches "transgenes Pflanzenmaterial" ist ein Pflanzenmaterial, das transgene Wirtszellen enthält, wobei die transgenen Wirtszellen in ihrem Genom mindestens ein Nukleinsäuremolekül stabil integriert haben, welches entweder heterolog ist in Bezug auf das Pflanzenmaterial oder welches normalerweise in nicht transformierten Zellen nicht vorhanden ist oder welches aber homolog ist in Bezug auf das Pflanzenmaterial, sich aber in den transgenen Zellen in einer anderen genomischen Umgebung befindet als in den Wildtypzellen.

In einer bevorzugten Ausführungsform enthält die transgene Pflanze einen Satz an Genen, der einen Teil der immunogenen Proteine des Säugervirus kodiert. Einer solchen Pflanze liegt die Idee zugrunde, dass die Pflanze nicht sämtliche immunogenen Epitope eines Erregers exprimiert, sondern lediglich eine Untergruppe, so dass nach Immunisierung mit der Untergruppe an Epitopen ein anderes Antikörpermuster erhalten wird, als nach Infektion des Organismus mit dem intakten Erreger. Anhand der unterschiedlichen Immunantworten zwischen immunisierten Organismen und infizierten Organismen können die immunisierten Organismen von den infizierten unterschieden werden.

Bei einer weiteren bevorzugten Ausführungsform wird die Pflanze ausgewählt aus Gerste, Weizen, Roggen, Triticale, Reis und Mais.

Vorzugsweise enthält die Pflanze Gene, welche für Proteine eines Pestivirus kodieren.

Es ist weiterhin bevorzugt, dass das Gen, das in den transgenen Pflanzen exprimiert wird, ein immunogenes Protein eines Virus kodiert, das ausgewählt ist aus dem Virus der Klassischen Schweinepest, dem Border Disease Virus und dem Virus der Bovinen Viralen Diarrhoe. Die Gene der Oberflächlichenproteine der genannten Viren sind bekannt und stehen dem Fachmann im Stand der Technik zur Verfügung.

Besonders bevorzugt zur Expression in der transgenen Pflanze ist das E2-Gen und das E^{ms}-Gen des Virus der Klassischen Schweinepest, wobei das Gen auch so modifiziert werden kann, beispielsweise durch gezielte Mutagenese, dass Derivate des natürlichen Genprodukts in der Pflanze hergestellt wurde. Dies kann z. B. vorteilhaft sein, um die Stabilität der Genprodukte positiv zu beeinflussen oder auch deren immunogenen Eigenschaften.

Damit eine möglichst hohe Expressionsausbeute an gewünschtem Genprodukt in der Pflanze erhalten wird, ist es von Vorteil, die Kodons der Gene so zu optimieren, dass sie zu maximalen Expressionsraten in der Pflanze führen. Solche Kodon-Optimierungen sind dem Fachmann für gegebene Wirte geläufig.

Bei einer weiteren bevorzugten Ausführungsform wird das gewünschte Genprodukt insbesondere im Getreidekorn der Pflanze exprimiert. Bei Einsatz eines entsprechenden Promotors, der z.B. unter Mälzungsbedingungen aktiviert wird, lässt sich somit das gewünschte Genprodukt zu einem beliebigen Zeitpunkt durch Induktion des entsprechenden Promotors induzieren.

Die vorliegende Erfindung betrifft ferner Tierfutter bzw. Nahrungsmittel, die eine Getreidepflanze oder Teile davon, wie oben beschrieben, enthalten. Diese Zusammensetzungen können dann z. B. bei oraler Verabreichung zu einer Stimulation des Immunsystems gegen entsprechende Viren führen. Insbesondere hat der Einsatz von Tierfutter entscheidende Vorteile gegenüber den bisher praktizierten Techniken der Impfung mittels Injektion, da die Verfütterung von Tierfutter wesentlich einfacher durchzuführen ist und auch z. B. zur Immunisierung von wild lebenden Tieren ohne weiteres möglich ist.

### Figurenkurzbeschreibung

- **Figur 1**: zeigt den Vektor pRS265.
- **Figur 2**: zeigt den Vektor pRS266.
- **Figur 3**: zeigt den Vektor pRS2600.
- **Figur 4**: zeigt den Vektor pRS268
- **Figur 5**: zeigt den Vektor pAM300
- **Figur 6**: zeigt den Vektor pRS269.

### Die folgenden Beispiele erläutern die Erfindung.

### 1. Chimäre Gene

Zur optimalen Ausbeute an Expressionsprodukt wird das Gen, das für ein Oberflächenprotein eines tierpathogenen Erregers kodiert, einer "Kodonoptimierung" unterzogen. Dabei werden Änderungen in der kodierenden Sequenz des Gens derart vorgenommen, dass die Kodons des Wildtyps durch die für die Wirtspflanze optimalen Kodons ersetzt werden, ohne die Aminosäuresequenz des kodierten Proteins zu verändern. In den Beispielen wurde die optimale Sequenz für Gerste ermittelt. Das "chimäre Gen" betrifft ein Gënkonstrukt, das eine DNA-Sequenz enthält, die für ein Fusionsprotein kodiert, wobei es eine (i) N-terminale Einheit einer Signalsequenz und einen (ii) unmittelbar an den C-terminalen Bereich der Aminosäuresequenz dieser Signalsequenz anschließenden Proteinanteil eines reifen heterologen Proteins enthält.

Die zur Expression eingesetzten immunogenen Strukturproteine stammen von Pestiviren, wobei sich der Genus *Pestivirus* im wesentlichen aus dem Virus der Klassischen Schweinepest (KSPV), dem Border Disease Virus (BDV) und dem Virus der Bovinen Viralen Diarrhoe (BVDV) zusammensetzt. Das Pestivirusgenom besteht aus einer (+)-Strang RNA von etwa 12,5 kb Länge mit einem einzelnen offenen Leserahmen, der für ein Polyprotein von ca. 4.000 Aminosäuren kodiert. Das Polyprotein wird ko- und postranslational von zellulären und viralen Proteasen prozessiert. Die viralen Strukturproteine, zu denen die Hüllproteine E2, E^{rns} und E1 gehören, liegen im N-terminalen Bereich des Polyproteins. Das native oder synthetische Gen kann im Hinblick auf eine erhöhte Expression des Gens oder Stabilität der exprimierten Proteine im Pflanzengewebe modifiziert sein. Zur Veränderung der Gensequenz kommen beispielsweise die gerichtete Mutagenese, Exon-shuffling und Ähnliches in Betracht.

Bei einem bevorzugten Konstrukt umfasst das chimäre Genkonstrukt 5' der kodierenden Region einen Promotor einer monokotyledonen Pflanze. Geeignete Promotoren sind der D-Hordein Promotor (SEQ ID NO: 3) und der Amylase Promotor (SEQ ID NO: 4) der Gerste, die z. B. auch in Whittier R.F. *et al*. (1987) Nucleotide sequence analysis of alpha-amylase and thiol protease genes that are hormonally regulated in barley aleurone cells. *Nucl. Acids Res*. **15**, 2515-2535 und Sørensen M.B. *et al.* (1996) Hordein promoter methylation and transcriptional activity in wildtype and mutant barley endosperm. *Mol*. *Gen. Genet*. **250,** 750-60 beschrieben sind.

Weiterhin enthält das chimäre Gen vorzugsweise zwischen dem Promotor und der kodierenden Sequenz die 5'-nicht-translatierte Region (5'-UTR) und 3' von der kodierenden Sequenz die nicht-translatierte Region (3'-UTR) eines pflanzlichen oder bakteriellen Gens. Eine bevorzugte 3'-UTR ist die nos-Terminator-Sequenz. Diese Sequenz umfasst die nicht-kodierende Sequenz 5' von der Polyadenylierungsstelle, die Polyadenylierungsstelle und die Transkriptions-Terminations-Sequenz und ist u. a. beschrieben in Jensen L.G. *et al*. (1998) Inheritance of a codon-optimized transgene expressing heat stable (1,3-1,4)-beta-glucanase in scutellum and aleurone of germinating barley. *Hereditas* **129**:215-225.

### 2. Pflanzentransformation

Im Rahmen der Transformation der Pflanzen wird das chimäre Gen der Erfindung in einen entsprechenden Expressionsvektor für Pflanzen eingebracht. Der Vektor enthält spezielle Elemente von Plasmiden oder Viren, die es ermöglichen, dass DNA von den Bakterien in die Pflanzen transportiert wird. Solche Expressionsvektoren für Pflanzen sind z.B. beschrieben in US 5,693,506.

### A. Transformationsvektoren

Die Vektoren enthalten neben den hierzu beschriebenen chimären Genen Selektions-Marker für den Gebrauch in Pflanzenzellen, z.B. das nptll Kanamycin-Resistenzgen oder das Phosphinotricin Acetytransferase Gen, sowie Sequenzen, die eine Selektion und ein Wachstum in sekundären Wirten, wie z.B. Hefe oder Bakterien, erlauben.

Darüber hinaus enthalten die hierzu verwendeten Vektoren Regionen, die zu einem Agrobakterium tumefaciens Vektor homolog sind, hier speziell die T-DNA Grenzsequenzen.

Transformations-Experimente mit sogenannten Doppelkassetten-Vektoren erlauben, das notwendige "Selektions-Gen" durch Auskreuzen vom gewünschten Transgen zu trennen. Dies ist vor allen Dingen für Proteine, die, wie im vorliegenden Projekt, oral appliziert werden sollen, von Bedeutung. Auf diesen Doppelkassetten-Vektoren werden sowohl das Selektions-Gen, z.B. das Bar-Gen, als auch das oben charakterisierte chimäre Gen jeweils von separaten T-DNA Grenzsequenzen flankiert, so dass die Wahrscheinlichkeit, dass es zu unabhängigen Rekombinationsereignissen ins Pflanzengenom kommt, gegeben ist.

### B. Transformation der Pflanzenzellen

Zur Transformation des Pflanzenmaterials kann vorzugsweise, aber nicht nur, nach der in US5,693,506 oder WO9,836,085 gegebenen Anleitung vorgegangen werden: Embryonen werden aus unreifem Samen präpariert und nach 48 Stunden Inkubation mit transformierten Agrobakterien ko-kultiviert. Es folgen verschiedene Waschschritte und Inkubation zunächst im Dunkeln, später im Licht.

Nach entsprechenden Selektionsschritten werden die ausgewählten Pflanzen angezogen und vermehrt. Die Expressionsrate des Fremd-Proteins wird zuerst im unreifen, später im reifen Korn determiniert.

### 3. Immunisierung der Tiere

Die Immunisierung der Tiere erfolgt bevorzugt bei Haus- oder Wildschweinen gegen eine virale Infektion, vorzugsweise die Klassische Schweinepest. Diese umfasst die Transformation von Pflanzenmaterial mit einem oben definierten Vektor, die Anzucht und Vermehrung der transformierten Pflanze, die Ernte des gewünschten Pflanzenmaterials sowie das anschließende, vorzugsweise mehrmalige Verfüttern des geernteten Materials, z.B. Gerstenkömer oder -malz, an die Tiere; dabei ist das Material entweder unbehandelt oder vermälzt. Alternativ kann das durch die DNA des Vektors kodierte Protein aus dem Pflanzenmaterial gereinigt und als Futterergänzung den Tieren verabreicht werden.

Die Immunisierung mittels der hierin beschriebenen Verfahren umfasst somit folgende Schritte:
(1) Konstruktion von Codon-optimierten Genen, die für immunogene Oberflächenproteine viraler Krankheitserreger kodieren;
(2) Transformation von Getreidepflanzen mit einem oder mehreren, aber nicht allen, Gen(en) für (ein) immunogene(s) Oberflächenprotein(e) viraler Krankheitserreger;
(3) Charakterisierung, Aufzucht und Vermehrung der transgenen Getreidepflanzen;
(4) Ernte der Körner;
(5a) Fütterung bzw. Zufütterung der Tiere mit den Körnem oder
(5b) Vermälzung der Gerstenkörner und Fütterung bzw. Zufütterung mit Malz.

### Kurze Beschreibung der Sequenzen

SEQ ID NO:1 ist die Aminosäure-Sequenz des E2-Strukturproteins des KSP-Virus
SEQ ID NO:2 ist die Nukleinsäure-Sequenz des Kodon-optimierten E2-Gens des KSP-Virus
SEQ ID NO:3 ist die Sequenz des D-Hordein-Promotors aus Gerste (Kornfüllung)
SEQ ID NO:4 ist die Sequenz des Amylase Promotors (Keimung)

### Beispiele

### Beispiel 1

### Konstruktion des Kodon-optimierten E2-Gens des KSP-Virus.

Die Kodons des Gens für das Strukturprotein E2 des KSP Virus werden in einer Weise optimiert, dass für die einzelnen Aminosäuren jeweils die in Frage kommenden GC-reichen Tripletts verwendet werden. Zu diesem Zweck wird das Gen aus insgesamt 56 Oligonukleotiden (54 mit einer Länge von jeweils 40 Nukleotiden und zwei mit einer Länge von 20 Nukleotiden) neu zusammengebaut: In den beiden Oligonukleotiden des oberen und unteren Stranges, die das Stop-Kodon beinhalten, ist direkt nach dem Stop-Codon eine *SacI* Restriktionsschnittstelle für spätere Klonierungsschritte eingefügt. Die Oligonukleotide werden mittels Ligation und PCR zusammengefügt.

Das Kodon-optimierte Gen wird durch eine "Splice-PCR" (siehe z. B. Horton *et al*., 1989, Gene **77**:61-68) hinter ein 3'-Fragment des Hordein-Promotors mit einem Hordein-Signal-Peptid gesetzt, das im vorderen Teil eine *Nco*I Schnittstelle beinhaltet.

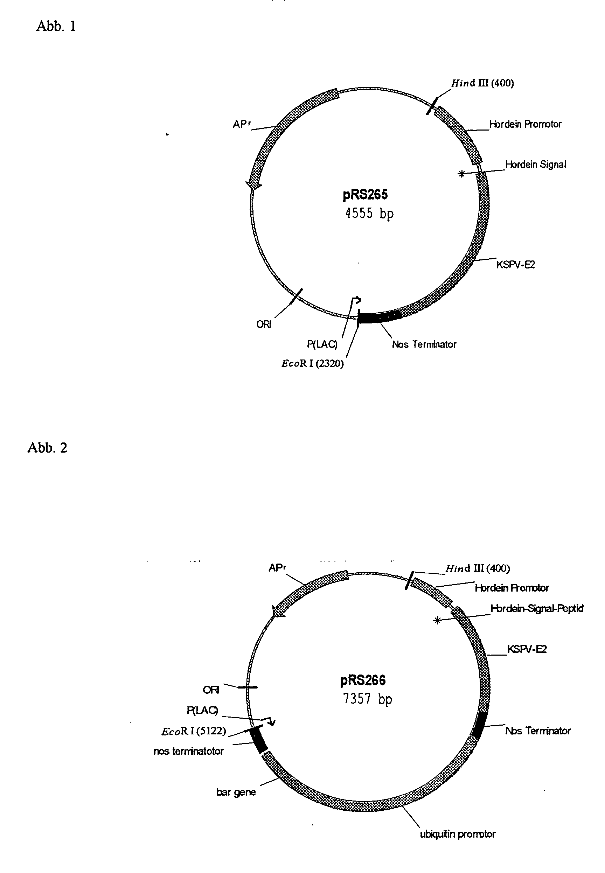

Nach Klonierung in einen pUC18-Vektor wird dieses Fragment (bestehend aus dem hinteren Teil des Hordein Promotors, dem Hordein-Signal-Peptid und dem vollständigen E2-Gen) über *Ncol* und Sacl-Restriktionsschnittstellen ausgeschnitten und in einen Vektor kloniert, der einen vollständigen Hordein-Promotor, ein Hordein-Signal-Peptid und einen Nos-Terminator enthält. Nach der Klonierung erhält man ein Konstrukt (RS265; Fig. 1), das aus Hordein-Promotor, Hordein-Signal-Peptid, E2-Gen und Nos-Terminator in pUC18 besteht.

### Beispiel 2

### Konstruktion KSP-Virus E2-exprimierender Vektoren als Einzel- und Doppelkassette

Ausgehend von Konstrukt RS265 werden nun zwei für die Transformation von Agrobakterium des Stammes Agll taugliche Vektoren zusammengesetzt.
Ein Konstrukt beinhaltet eine sogenannte Einzelkassette, das andere eine Doppelkassette, die sich dahingehend von der Einzelkassette unterscheidet, dass die beiden chimären Gene (1) Ubiquitin-Promotor, Markergen, Nos-Terminator und (2) Hordein-Promotor, Hordein-Signal-Peptid, E2-Gen, Nos-Terminator jeweils von einer linken und einer rechten T-DNA Grenzregion umgeben sind, um eine spätere unabhängige Integration ins Pflanzengenom zu ermöglichen. Bei der Einzelkassette ist die gesamte Einheit aus Ubiquitin-Promotor, Markergen, Nos-Terminator und Hordein-Promotor, Hordein-Signal-Peptid, E2-Gen, Nos-Terminator nur von jeweils einer gemeinsamen T-DNA Grenzregion eingefaßt.

### Konstruktion der Einzelkassette:

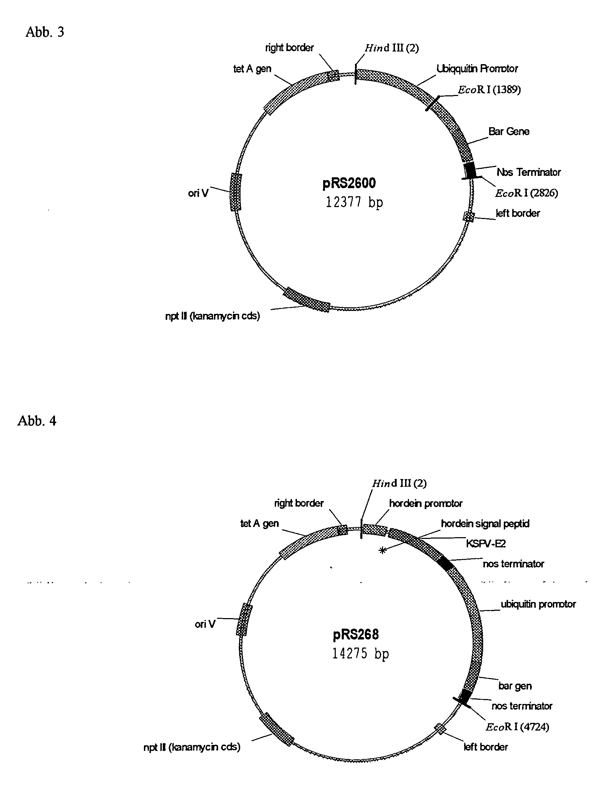

Das oben beschriebenen Konstrukt RS265 wird zunächst mit *Eco*RI gespalten, dann werden die überhängenden Enden durch eine T4 DNA Polymerase Behandlung aufgefüllt und anschließend eine *Hind*III-Spaltung durchgeführt. Das gereinigte Fragment, auf dem das E2-Gen lokalisiert ist, wird in einen pUC18 Vektor ligiert, der eine Einheit bestehend aus Ubiquitin-Promotor, Markergen und Nos-Terminator trägt. Dieser Vektor wird vor der Ligation mit *Bam*HI und *Hind*III gespalten, und auch hier wird vor der *Hind*III Spaltung die *Bam*HI Restriktionsschnittstelle aufgefüllt. Nach dieser Klonierung erhält man den Vektor RS266 (Fig. 2), aus dem die gesamte Einheit bestehend aus Ubiquitin-Promotor, Markergen, Nos-Terminator und Hordein Promotor, Hordein Signal Peptid, E2-Gen des KSP-Virus, Nos-Terminator mittels *Eco*RI/*Hind*III ausgeschnitten und in eine entsprechend geschnittenen pRS2600-Vektor (Fig. 3) hineingesetzt wird. Mit diesem Konstrukt RS268 (Fig. 4) werden mittels Elektroporation Agrobakterien des Stammes Agll transformiert.

### Konstruktion der Doppelkassette:

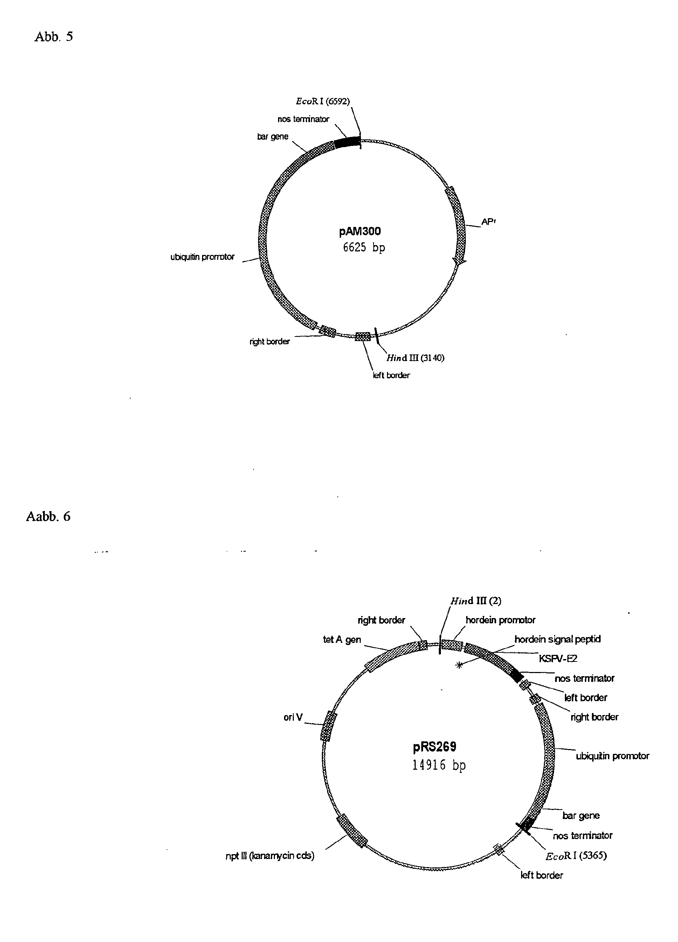

Aus dem oben beschriebenen Konstrukt RS265 wird mittels *Hind*III und *Eco*RI das Fragment mit dem E2-Gen herausgeschnitten, wobei wiederum, wie oben beschrieben, die *Eco*RI Restriktionsschnittstelle vor der *Hind*III-Spaltung aufgefüllt wird. Der speziell für die Erstellung von Doppelkassetten konstruierte Vektor pAM300 (Fig. 5) beinhaltet bereits je eine linke und rechte T-DNA Grenzregion, sowie eine Einheit bestehend aus Ubiquitin-Promotor, Markergen und Nos-Terminator. Dieser Vektor wird mit *Not*I/*Hind*III geschnitten, wobei hier die *Not*I-Schnittstelle aufgefüllt wird. Anschließend wird das *Hind*III/*Eco*RI Fragment aus dem RS265 in diesen Vektor kloniert. Danach wird eine Einheit bestehend aus Ubiquitin-Promotor, Markergen, Nos-Terminator und Hordein Promotor, Hordein Signal Peptid, E2-Gen, Nos-Terminator, getrennt durch je eine linke und rechte Grenzsequenz mittels *Eco*RI und *Hind*III ausgeschnitten und in den entsprechend geschnittenen pRS2600 hineingesetzt.
Mit diesem Konstrukt RS269 (Fig. 6) werden mittels Elektroporation Agrobakterien des Stammes Agll transformiert.

### Beispiel 3

### Agrobakterium-vermittelte Transformation der Gersten-Linie Golden promise

Gerstenpflanzen - vorzugsweise der Varietät *Golden promise -* werden z.B. in Klimakammem herangezogen. Nach ca. 2-3 Monaten können unreife Gerstensamen, deren Embryonen einen Durchmesser von ca. 1,5 mm - 2,5 mm haben, geemtet werden.

Die Gerstensamen werden sterilisiert, nach dem Waschen aus den Körnern herauspräpariert und in Längsrichtung geteilt. Unter Umständen kann es hier sinnvoll sein, die Embryonen vor dem Herauspräparieren zu teilen, indem einfach das Samenkorn geteilt wird. Der geteilte Embryo wird in diesem Fall nach dem Schneiden herauspräpariert. Die Embryonen werden 48 h bei 24° C inkubiert.

Agrobakterien z. B. des Stammes Agll, die mit den oben beschriebenen Transformationsvektoren transformiert worden sind, werden 48 h bei Raumtemperatur in Selektions-Medium angezogen. Nach der Inkubation wird das Pflanzenmaterial mit der Bakteriensuspension infiziert. Die Inkubationszeit der Agrobakterien mit den Gerstenembryonen bei 24°C in einem belüfteten Inkubationsraum richtet sich nach dem Wachstum der Agrobakterien, beträgt aber in der Regel 48 h - 72 h.

Nach der Ko-Kultivierung werden die Gerstenembryonen mehrmals gewaschen, bis keine Trübung des Mediums durch Bakterien mehr sichtbar ist. Nach dem Umsetzen der Embryonen auf die Selektions-Platten werden diese in einem belüfteten Inkubationsraum im Dunkeln für zwei Wochen bei 24°C inkubiert. Die Selektionsplatten enthalten Wachstumshormon und ein Selektionsmittel, z.B. Bialaphos. Nach 2 Wochen werden die Embryonen auf neue Platten umgesetzt und lediglich Sprosse und Wurzeln großzügig entfernt. Die Platten werden wiederum zwei Wochen in Dunkeln bei 24°C inkubiert.

Nach zwei Wochen werden die Embryonen wiederum umgesetzt und anschließend unter speziell für Pflanzen geeignetem Licht gehalten. In dieser Phase sollen die Kalli grüne Bereiche entwickeln. Wenn die Pflänzchen groß genug sind, werden sie in Schalen umgesetzt, um Wurzeln auszubilden.

## Patentansprüche

1. Verfahren zum Herstellen eines Markerimpfstoffs gegen ein Säugervirus, **dadurch gekennzeichnet, dass** das bzw. die immunogenen Proteine des Impfstoffs von einer Getreidepflanze oder Teilen davon hergestellt werden.

2. Verfahren nach Anspruch 1, **dadurch gekennzeichnet, dass** ein Satz Gene in der Pflanze exprimiert wird, der einen Teil der immunogenen Proteine, aber nicht sämtliche immunogenen Proteine des Säugervirus kodiert.

3. Verfahren nach einem der Ansprüche 1 oder 2, **dadurch gekennzeichnet, dass** die Getreidepflanze ausgewählt wird aus Gerste, Weizen, Roggen, Triticale, Reis und Mais.

4. Verfahren nach einem der Ansprüche 1 bis 3, **dadurch gekennzeichnet, dass** das Säugervirus aus der Familie der Pestiviren stammt.

5. Verfahren nach einem der Ansprüche 1 bis 4, **dadurch gekennzeichnet, dass** Virus ausgewählt wird aus dem Virus der klassischen Schweinepest, dem Border Disease Virus und dem Virus der Bovinen Viralen Diarrhoe.

6. Verfahren nach einem der Ansprüche-1 bis 5, **dadurch gekennzeichnet, dass** das immunogene Protein ausgewählt wird aus dem Genprodukt des E2-Gens und/oder des E^{rns}-Gens des Virus der klassischen Schweinepest oder Derivaten davon.

7. Verfahren nach einem der Ansprüche 1 bis 6, **dadurch gekennzeichnet, dass** das Gen bzw. die Gene Kodon-optimiert für Getreidepflanzen sind.

8. Transgene Getreidepflanze oder Teile davon, enthaltend mindestens ein Gen, das für ein immunogenes Protein eines Säugervirus kodiert.

9. Transgene Pflanze nach Anspruch 8, **dadurch gekennzeichnet, dass** sie einen Satz an Genen enthält, der für einen Teil der immunogenen Proteine des Säugervirus kodiert.

10. Transgene Pflanze nach Anspruch 8 oder 9, **dadurch gekennzeichnet, dass** sie ausgewählt wird aus Gerste, Weizen, Roggen, Triticale, Reis und Mais.

11. Transgene Pflanze nach einem der Ansprüche 8 bis 10, **dadurch gekennzeichnet, dass** das Säugervirus ein Pestivirus ist.

12. Transgene Pflanze nach Anspruch 11, **dadurch gekennzeichnet, dass** das Säugervirus ausgewählt wird aus dem Virus der klassischen Schweinepest, dem Border Disease Virus und dem Virus der Bovinen Viralen Diarrhoe.

13. Transgene Getreidepflanze nach einem der Ansprüche 8 - 12, **dadurch gekennzeichnet, dass** das immunogene Protein das Genprodukt des E2-Gens und/oder des E^{ms}-Gens des Virus der klassischen Schweinepest oder ein Derivat davon ist.

14. Transgene Pflanze nach Anspruch 13, **dadurch gekennzeichnet, dass** das bzw. die Gen/e Kodon-optimiert für Getreidepflanzen ist/sind.

15. Transgene Pflanze nach einem der Ansprüche 8 bis 14, **dadurch gekennzeichnet, dass** das Gen im Getreidekorn exprimiert wird.

16. Tierfutter bzw. Nahrungsmittel, enthaltend eine Getreidepflanze oder Teile davon nach einem der Ansprüche 8 - 15.

17. Verwendung eines Markerimpfstoffs wie nach einem der Verfahren 1 bis 7, hergestellt, oder von Tierfutter nach Anspruch 16 zur Immunisierung von Säugern, insbesondere Schweinen.

18. Verwendung nach Anspruch 17, **dadurch gekennzeichnet, daß** der Markerimpfstoff oral verabreicht wird.


SEQUENZPROTOKOLL

<110> Maltagen Forschung GmbH, Andernach

<120> Verfahren zum Herstellen eines Markerimpfstoffs gegen
      ein Säugervirus

<130> P33126-031

<140>
<141>

<160> 4

<170> PatentIn Ver. 2.1

<210> 1
<211> 371
<212> PRT
<213> Virus der Klassischen Schweinepest

<220>
<223> E2-Protein des Virus der Klassischen Schweinepest

<400> 1
Arg Leu Ala Cys Lys Glu Asp Tyr Arg Tyr Ala Ile Ser Ser Thr Asn
  1               5                  10                  15

Glu Ile Gly Leu Leu Gly Ala Glu Gly Leu Thr Thr Thr Trp Lys Glu
             20                  25                  30

Tyr Ser His Gly Leu Gln Leu Asp Asp Gly Thr Val Lys Ala Val Cys
         35                  40                  45

Thr Ala Gly Ser Phe Lys Val Thr Ala Leu Asn Val Val Ser Arg Arg
     50                  55                  60

Tyr Leu Ala Ser Leu His Lys Arg Ala Leu Pro Thr Ser Val Thr Phe
 65                  70                  75                  80

Glu Leu Leu Phe Asp Gly Thr Asn Pro Ala Ile Glu Glu Met Asp Asp
                 85                  90                  95

Asp Phe Gly Phe Gly Leu Cys Pro Phe Asp Thr Ser Pro Val Ile Lys
            100                 105                 110

Gly Lys Tyr Asn Thr Thr Leu Leu Asn Gly Ser Ala Phe Tyr Leu Val
        115                 120                 125

Cys Pro Ile Gly Trp Thr Gly Val Val Glu Cys Thr Ala Val Ser Pro
    130                 135                 140

Thr Thr Leu Arg Thr Glu Val Val Lys Thr Phe Arg Arg Asp Lys Pro
145                 150                 155                 160

Phe Pro His Arg Val Asp Cys Val Thr Thr Ile Val Glu Lys Glu Asp
                165                 170                 175

Leu Phe His Cys Lys Leu Gly Gly Asn Trp Thr Cys Val Lys Gly Asp
            180                 185                 190

Pro Val Thr Tyr Lys Gly Gly Gln Val Lys Gln Cys Arg Trp Cys Gly
        195                 200                 205

Phe Glu Phe Lys Glu Pro Tyr Gly Leu Pro His Tyr Pro Ile Gly Lys
    210                 215                 220

Cys Ile Leu Thr Asn Glu Thr Gly Tyr Arg Val Val Asp Ser Thr Asp
225                 230                 235                 240

Cys Asn Arg Asp Gly Val Val Ile Ser Thr Glu Gly Glu His Glu Cys
                245                 250                 255

Leu Ile Gly Asn Thr Thr Val Lys Val His Ala Leu Asp Glu Arg Leu
            260                 265                 270

Gly Pro Met Pro Cys Arg Pro Lys Glu Ile Val Ser Ser Glu Gly Pro
        275                 280                 285

Val Arg Lys Thr Ser Cys Thr Phe Asn Tyr Thr Lys Thr Leu Arg Asn
    290                 295                 300

Lys Tyr Tyr Glu Pro Arg Asp Ser Tyr Phe Gln Gln Tyr Met Leu Lys
305                 310                 315                 320

Gly Glu Tyr Gln Tyr Trp Phe Asn Leu Asp Val Thr Asp His His Thr
                325                 330                 335

Asp Tyr Phe Ala Glu Phe Val Val Leu Val Val Val Ala Leu Leu Gly
            340                 345                 350

Gly Arg Tyr Val Leu Trp Leu Ile Val Thr Tyr Ile Ile Leu Thr Glu
        355                 360                 365

Gln Leu Ala
    370

<210> 2
<211> 1120
<212> DNA
<213> Virus der Klassischen Schweinepest

<220>
<223> Kodon-optimiertes E2-Gen des Virus der Klassischen
      Schweinepest

<400> 2
cgcctcgcct gcaaggagga ctaccggtac gcgatcagca gcaccaacga gatcggcctg 60
ctcggcgccg aggggctgac cacgacctgg aaggagtact cccacggcct ccagctggac 120

gacggcacgg tcaaggcggt gtgcaccgcc gggagcttca aggtcaccgc gctcaacgtg 180
gtctcgcgcc ggtacctggc cagcctccac aagcgcgccc tgccgacgag cgtgaccttc 240
gagctgctgt tcgacggcac caaccccgcc atcgaggaga tggacgacga cttcggcttc 300
gggctctgcc cgttcgacac gtcccccgtc atcaagggca agtacaacac caccctgctc 360
aacgggtcgg cgttctacct ggtgtgcccg atcggctgga cgggcgtcgt ggagtgcacc 420
gccgtcagcc ccaccacgct ccggaccgag gtggtcaaga cgttccgccg ggacaagccg 480
ttcccccacc gcgtggactg cgtcaccacg atcgtggaga aggaggacct gttccactgc 540
aagctcgggg gcaactggac ctgcgtcaag ggcgacccgg tgacgtacaa ggggggccag 600
gtcaagcagt gccggtggtg cggcttcgag ttcaaggagc cctacgggct gccgcactac 660
cccatcggca agtgcatcct caccaacgag accggctacc gcgtggtcga ctccacggac 720
tgcaaccggg acggggtggt catctcgacc gagggcgagc acgagtgcct gatcggcaac 780
accacggtga aggtccacgc gctcgacgag cgcctgggcc cgatgccctg ccggccgaag 840
gagatcgtgt cctccgaggg ccccgtccgc aagacctcgt gcaccttcaa ctacacgaag 900
accctccgga acaagtacta cgagccgcgc gacagctact tccagcagta catgctgaag 960
ggggagtacc agtactggtt caacctcgac gtgaccgacc accacacgga ctacttcgcc
1020
gagttcgtcg tgctggtcgt ggtcgcgctc ctgggcggcc ggtacgtgct ctggctgatc
1080
gtcacctaca tcatcctcac cgagcagctg gcctgagctc
1120

<210> 3
<211> 434
<212> DNA
<213> Hordeum vulgare

<220>
<223> Hordein-Promotor inkl. Sequenz für das Signalpeptid

<400> 3
cttcgagtgc ccgccgattt gccagcaatg gctaacagac acatattctg ccaaaacccc 60
agaacaataa tcacttctcg tagatgaaga gaacagacca agatacaaac gtccacgctt 120
cagcaaacag taccccagaa ctaggattaa gccgattacg cggctttagc agaccgtcca 180
aaaaaactgt tttgcaaagc tccaattcct ccttgcttat ccaatttctt ttgtgttggc 240
aaactgcact tgtccaaccg attttgttct tcccgtgttt cttcttaggc taactaacac 300
agccgtgcac atagccatgg tccggaatct tcacctcgtc cctataaaag cccagccaat 360
ctccacaatc tcatcatcac cgagaacacc gagaaccaca aaactagaga tcaattcatt 420
gacagtccac cgag 434

<210> 4
<211> 777
<212> DNA
<213> Hordeum vulgare

<220>
<223> Amylase-Promotor inkl. Sequenz für das Signalpeptid

<400> 4
ttcacacatt caaaaaaagc aaatttcaaa atttgtactc gaatttaaaa aaagatcatt 60
gatttaaaaa atttcgctaa gtaaaaaact gtccacgtat ttcaaaaaaa gataagtggg 120
catcaatcgg caaaatttga tatgtcaggg tattgagcca tattaatttg gttgtggcac 180
aatatttttg tcccccgttg caacgcacgg ttatttttgt tagtgtgttc ttatggatat 240
atacgcccat aaagtttaag caatacaaca actatttcac caatccctct attctctttc 300
ttcacagcag atagaaggtt ttgtaattgt aaccacagca cactattcga tgaaaaatgc 360
atcgaatgtt ctgtcctcag aaaaacagag gttgaggata actgacggtc gtattgatcc 420
ggtgccttct tatggaaggc caaggctgcc tccatctaca tcacttggga cattgaatcg 480

ccttttgagc tcaccgtacc ggccgataac aaactccggc tgacatatcc actggcccaa 540
aggagcattg aagccgagca cacgagaaag tgatttgcaa gttgcacacc ggcagcaatt 600
ccggcatgct gcagcacact ataaatacct ggccagacac acaagttgaa tgcatcagtt 660
ctccatcgta ctcttcgaga gcacagcaag agagagctga agaacatggc gaacaaacat 720
ttgtccctct ccctcttcct cgtcctcctt ggcctgtcgg ccagcttggc ctccggg 777